# EUROPEAN PATENT APPLICATION

(11) **EP 1 821 104 A2**
(43) Date of publication of application: **22.08.2007**
(21) Application number: 07007737.5
(22) Date of filing: 08.02.2001
(51) Int. Cl.: G01N 33/68

(54) **Method for diagnosing efficacy of xenotypic antibody therapy**

(30) Priority: 08.02.2000 US 181008 P; 04.05.2000 US 201868 P
(62) Divisional of application: 01912066.6
(71) Applicant: AltaRex Medical Corporation, Edmonton Alberta T6E 6S4 (CA)
(72) Inventor: Noujaim, Antoine, Edmonton, Alberta T6M 2K4 (CA)
(74) Representative: Chapman, Paul Gilmour

(57) **Abstract**

The invention provides methods for diagnosing the efficacy of a patient to xenotypic antibody therapy which include (1) measuring the level of an antibody produced by a patient that specifically binds to a xenotypic antibody after administration of the xenotypic antibody to the patient; (2) measuring the level of an anti-idiotype antibody produced by a patient that specifically binds to a xenotypic antibody after administration of the xenotypic antibody to the patient; (3) measuring the level of an antibody produced by a patient that specifically binds to a target antigen of a xenotypic antibody after administration af a xenotypic antibody to the patient; and (4) measuring the level of a T cell response produced by a patient to a target antigen of the xenotypic antibody after administration of a xenotypic antibody to the patient. In the methods of the invention, an increase in the level of antibody or T cell response produced by the patient after the administration of the xenotypic antibody relative to the level antibody or T cell response produced by the patient prior to the administration of the xenotypic antibody is indicative of a favorable diagnosis of efficacy.

## Description

### CROSS-REFERENCE TO RELATED APPLICATIONS

This application claims benefit from U.S. Provisional Patent Application Serial No. 60/181,008 filed February 8, 2000, and U.S. Provisional Patent Application Serial No.60/201,868, filed May 4, 2000; the entire contents of which are hereby incorporated by reference.

### BACKGROUND OF THE INVENTION

### Field of the invention

The invention relates to xenotypic antibody-mediated immunotherapy.

### Summary of the related art

Xenotypic antibody-mediated immunotherapy is an emerging therapeutic approach for a variety of diseases. Ongoing clinical trials utilize murine monoclonal antibodies directed against CA125 antigen to treat ovarian cancer in humans. Ovarian cancer patients in traditional therapies have a high frequency of short-term relapse. Unfortunately, relapse is commonly not detected until the reappearance of CA125 antigen in the patient's blood stream. By that time, medical intervention options may be more limited than they might have been if the relapse could have been predicted earlier.

There is therefore a need for a method for predicting the likelihood of success of xenotypic antibody-mediated immunotherapy.

### BRIEF SUMMARY OF THE INVENTION

The invention provides a method for predicting the likelihood of success of xenotypic antibody-mediated immunotherapy. The invention further provides a method for diagnosing the period of time after xenotypic antibody-mediated immunotherapy during which a patient will be free from relapse.

The present inventor has surprisingly discovered that patients who receive xenotypic antibody-mediated immunotherapy have a much higher likelihood of success (longer period of avoiding relapse) if the patient produces high levels of anti-xenotypic antibody upon initial treatment. Note that the where the patient is a human, the human anti-xenotypic antibody response is abbreviated HAXA.

Thus, the invention provides a method for diagnosing the efficacy of xenotypic antibody-mediated immunotherapy, the method comprising measuring the level of anti-xenotypic antibody *(e.g.,* HAXA) produced by the patient after administration to the patient of xenotypic antibody. In preferred embodiments, an increase in the level of anti-xenotypic antibody produced by the patient after administration of the xenotypic antibody relative to the level of anti-xenotypic antibody produced by the patient prior to administration of the xenotypic antibody is indicative of a favorable diagnosis of efficacy.

The invention further provides a method for diagnosing the efficacy of xenotypic antibody-mediated immunotherapy, the method comprising measuring the level of anti-idiotype antibody (Ab2) produced in response to xenotypic antibody administration An "anti-idiotype antibody" means an antibody that specifically binds to the variable region of an antibody, thus partially or completely blocking the ability to the xenotypic antibody to specifically bind to its epitope on the target antigen *(e.g.,* an anti-idiotype antibody that specifically binds to an administered xenotypic antibody specifically binds to the variable region of the xenotypic andobdy).

The invention further provides a method for diagnosing the efficacy of xenotypic antibody-mediated immunotherapy, the method comprising measuring the level of antibody to the target antigen of the xenotypic antibody produced in response to xenotypic antibody administration.

The invention further provides a method for diagnosing the efficacy of xenotypic antibody-mediated immunotherapy, the method comprising measuring the level of T cell stimulation response to the target antigen of the xenotypic antibody produced in response to xenotypic antibody administration. In certain embodiments, the T cell response is a helper T cell response, a cytotoxic T cell response, or a combination of helper and cytotoxic T cell responses.

### BRIEF DESCRIPTION OF THE DRAWINGS

Figure 1 shows the relationship of median time to relapse to different values of HAMA used for the definition of responder.
Figure 2 shows the correlation between level of HAMA and level of Ab2.
Figure 3 shows correlation between increased level of Ab2 and increased survival.
Figure 4A shows the correlation between level of HAMA and level of anti-CA125 antibody (*i.e.,* antibody specific to CA125 antigen) produced by the patient.
Figure 4B shows the correlation between the level of HAMA and the increase in level of anti-CA125 antibody produced by the patient.
Figure 5 shows correlation between anti-CA125 antibody and survival.
Figure 6 shows correlation between increased level of T cell stimulation and increased survival.

### DETAILED DESCRIPTION OF THE EMBODIMENTS

The invention relates to xenotypic antibody-mediated immunotherapy. The invention provides a method for predicting the likelihood of success of xenotypic antibody-mediated immunotherapy. Accordingly, using the methods of the invention, an assessment may be made as to whether xenotypic antibody-mediated immunotherapy is efficacious and thus should be continued in the patient, or whether the xenotypic antibody-mediated is not efficacious, and the patient should thus be re-evaluated for a possible alternate treatment. The invention further provides a method for diagnosing the period of time after xenotypic antibody-mediated immunotherapy during which a patient will be free from relapse.

The present inventor has surprisingly discovered that patients who receive xenotypic antibody-mediated immunotherapy have a much higher likelihood of success (longer period of avoiding relapse) if the patient develops high levels of human anti-xenotypic antibodies (HAXA) upon initial treatment.

Thus, in a first aspect, the invention provides a method for diagnosing the efficacy of xenotypic antibody-mediated immunotherapy, the method comprising measuring the level of HAXA produced by the patient after administration to the patient of xenotypic antibody. In preferred embodiments, an increase in the level of anti-xenotypic antibody produced by the patient after administration to the patient of the xenotypic antibody relative to the level of anti-xenotypic antibody produced by the patient prior to administration of the xenotypic antibody is indicative of a favorable diagnosis of efficacy.

As used herein, "diagnosing the efficacy" means predicting the time after administration of a xenetypic antibody at which relapse occurs. By "favorable" diagnosis" is meant a diagnosis that predicts that the time after administration of a xenotypic antibody at which relapse occurs is longer than the time after administration of a placebo (*e.g.*, sugar solution or physiological saline solution) at which relapse occurs. In all of the aspects of the invention, a favorable diagnosis of efficacy increases the time to disease progression or increases the likelihood of survival of the patient.

As used herein, "efficacy" means having the ability to delay disease progression or extend the life of a diseased patient. "Relapse" means the return of clinically observable signs or symptoms of disease. "Xenotypic antibody-mediated immunotherapy" means the administration of an antibody from one species of animal to a second species of animal having a disease, where the antibody forms an antibody-antigen pair with an antigen in the body of the second species that is associated with the disease, thereby reducing or eliminating clinically relevant signs or symptoms of the disease, as may be determined by any ordinarily skilled health care professional *(e.g.,* a nurse or a physician). By "target antigen associated with the disease" is meant an antigen which is found in greater quantities or as a altered protein in patients suffering from a disease. Non-limiting examples of target antigens associated with disease are CA125, which is associated with ovarian cancer, and prostate specific antigen, which is associated with prostate cancer.

As used herein, "HAXA" means a human antibody response against a xenotypic antibody, where the human has been administered the xenotypic antibody. "Xenotypic antibody" means antibody from another species. (Note that "antibody" and "antibodies" are used interchangeably throughout). Thus, if the patient is a human, the xenotypic antibodies would be non-human antibodies. As used herein, by "administer" or "administering" or "administration" is meant delivery of a xenotypic antibody by any suitable means, including, without limitation, intramuscular administration, intradermal, intravenous, intra-arterial peritoneal, subcutaneous, and intra-lymphatic. Those of ordinary skill in the art will realize that a xenotypic antibody can be administered according to the methods of the invention in any physiologically acceptable formulation *(e.g.,* with saline solution). Methods for making pharmaceutically acceptable carriers and formulations thereof are found, for example, in Remington's Pharmaceutical Sciences (18th edition), ed. A. Gennaro (1990) Mack Publishing Company, Easton, PA.

Preferred diseases treated by xenotypic antibody-mediated immunotherapy include cancers, inflammatory diseases, and bacterial, parasitic, and viral infections. Particularly preferred are ovarian cancer, breast cancer and prostate cancer. Preferred xenotypic antibodies include, without limitation, murine monoclonal antibodies. Particularly preferred antibodies include, without limitation, OvaRex^{™} (which specifically binds to the CA,125 antigen BrevaRex^{™} (which specifically binds to the MUC-1 antigen) and ProstaRex^{™} (which specifically binds to prostate specific antigen). In certain preferred embodiments, the HAXA response comprises human antibodies that specifically bind to any portion of the complementarity determining region of the xenotypic antibody. In certain embodiments, the HAXA response comprises an anti-idiotype antibody that specifically binds to the variable region of the administered xenotypic antibody, thus partially or completely blocking the ability to the xenotypic antibody to specifically bind to its epitope on the target antigen. As used herein, by "specifically bind" is meant that an antibody recognizes and binds to a particular target antigen (*i.e.,* its target antigen), but does not substantially recognize and bind to other molecules in a sample, *e.g.*, a biological sample that naturally includes many different proteins. Preferably, the antibody specifically binds its target antigen at a site on the target antigen called an epitope. The association formed between the binding agent and its ligand may be covalent, and is preferably non-covalent Preferably, a binding agent that specifically binds to its target antigen forms an association with that target antigen with an affinity of at least 10⁶M⁻¹, more preferably, at least 10⁷M⁻¹, even more preferably, at least 10⁸ M⁻¹, and most preferably, at least 10⁹M⁻¹ either in water, under physiological conditions, or under conditions which approximate physiological conditions with respect to ionic strength, *e.g.,* 140 mM NaCl, 5 mM MgCl₂.

In certain preferred embodiments, the HAXA response comprises human antibodies that bind the non-complementarity determining region of the xenotypic antibody. In certain preferred embodiments, the level of HAXA rises to more than 100-fold the level that was present before the administration of xenotypic antibody. In certain preferred embodiments, the level of HAXA rises to more than 3-fold the level that was present before the administration of xenotypic antibody within two weeks of administration of xenotypic antibody. In certain preferred embodiments, the level of HAXA rises to more than 2-fold the level that was present before the administration of xenotypic antibody after at least 3 injections of xenotypic antibodies. In certain preferred embodiments, the HAXA response is at least 200 ng antibody/ml blood. In certain preferred embodiments, the HAXA response is at least 5,000 ng antibody/ml blood. In certain preferred embodiments, the HAXA response is at least 10,000 ng antibody/ml blood. In certain preferred embodiments, the HAXA response is at least 40,000 ng antibody/ml blood.

In certain preferred embodiments, the HAXA response causes the body to make antibodies (Ab3) that can compete with the xenotypic antibody for binding its target antigen (i.e., the Ab3 antibody specifically binds to a region on the target antigen that completely or partially blocks the ability of the xenotypic antibody to specifically bind to its epitope on the target antigen). In certain preferred embodiments, the Ab3 is present at least 3-fold higher than the level of Ab3 present before the administration of xenotypic antibody.

In preferred embodiments, the HAXA response increases the time to disease progression. Thus, if there is a HAXA response, the disease progresses more slowly. In preferred embodiments, the HAXA response results in an increase in survival.

In certain preferred embodiments, administration of the xenotypic antibody results in a T-cell response against the target antigen of the xenotypic antibody that has a stimulation index of greater than 1.5 times higher than before the administration of the xenotypic antibody. The stimulation index can be determined according to standard T cell stimulation assays *(e.g.,* 1.5 times higher ³H-thymidine uptake by T cells proliferating in the presence of the target antigen as compared to the ³H-thymidine uptake by T cells proliferating in the absence of the target antigen).

In one non-limiting example of the methods of the invention, human patients with ovarian cancer were administered a murine monoclonal antibody that specifically binds a target antigen associated with ovarian cancer, and then tested to determine if they had produced HAMA in response to the murine antibody. In this non-linniting example, ovarian cancer patients were administered a murine monoclonal anti-CA125 antibody at a dosage of 1 mg/kg body weight. Later, serum samples were tested for the presence of human anti-mouse antibodies (HAMA). In the analysis of the test results of the serum samples, a "HAMA responder" was taken to be a treated patient with a substantial antibody response, defined as an on-study maximum HAMA response greater than or equal to 10,000 ng/mL. An analysis of the time to relapse was undertaken using HAMA responder/HAMA non-responder as a stratification factor. The results are shown in Table I below. There were 44 patients in the antibody treatment group who were considered HAMA responders using the above criterion. The median time to relapse for these patients *(i.e.,* the HAMA responder patients) was 16.38 months, compared to 7.76 months for antibody treated HAMA non-responders and 11.34 months for placebo control patients. These results demonstrate that significant HAMA response is strongly predictive of time to relapse.

**Table I: Time to relapse - Kaplan-Meier analysis**

| Parameter | HAMA responders | HAMA non-responders | Placebo |
|---|---|---|---|
| Number of patients | 44 | 81 | 126 |
| Relapsed N (%) | 19(43.2%) | 46 (56.8%) | 62 (49.2%) |
| Censored N (%) | 25(56.8%) | 35(43.2%) | 64 (50.8%) |
| Time to Relapse | | | |
| 25th percentile | 7.66 (6.97,12.53) | 3.62 (2.47, 4.93) | 5.49.(4.64,7.43) |
| Median (95% Confidence Interval) | 16.38 (11.97) | 7.76(6.02,19.20) | 11.34(9.87,19.89) |
| 75th percentile | (18.91) | (19.20) | (19.89) |

In a follow-up analysis of these patients, a "HAMA responder" was taken to be an OvaRex^{™}-treated patient with a substantial antibody response, in this case ≥ 5,000 ng/mL. An analysis of the time to relapse was undertaken using HAMA responder/ non-responder as a stratification factor. The results are shown in Table II below.

**Table II: Time to relapse - Kaplan-Meier analysis**

| Parameter | HAMA responders | HAMA non-responders | Placebo |
|---|---|---|---|
| Number of patients | 64 | 61 | 126 |
| Relapsed N(%) | 27 (422%) | 38(62.3%) | 62 (49.2%) |
| Censored N(%) | 37 (57.8%) | 23 (37.7%) | 64(50.8%) |
| Time to Relapse | | | |
| 25th percentile | 7.66 (7.37,10.10) | 2.66 (1.84, 444) | 5.49 (4.64,7.43) |
| Median (95% Confidence Interval) | 16.38 (10-10) | 6.51 (4.51,12.27) | 11.34 (9.87, 19.89) |
| 75th percentile | (18.91) | (12.27) | (19.89) |

As shown in Table II, there were 64 patients in the OvaRex^{™} treatment who were considered HAMA responders using the above criterion. The median time to relapse for these patients was 16.38 months, compared to 6.51 months in the OvaRex^{™} treatment HAMA non-responder group and 11.34 months in the placebo group.

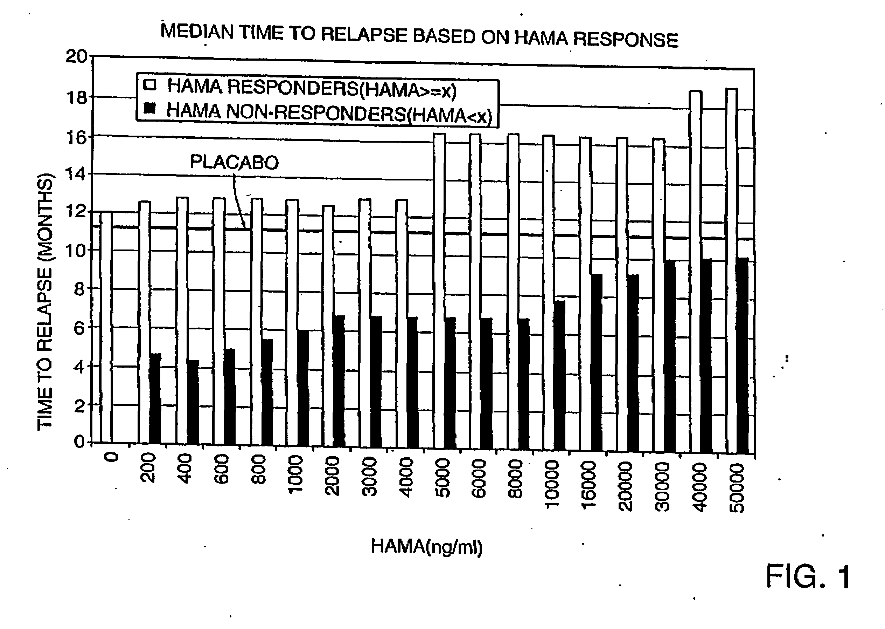

Figure 1 show the relationship of median time to relapse to different values of HAMA used for the definition of responder. As Figure 1 shows, there is a slight increase in median time to relapse for HMA responders defined as patients with HAMA of at least 200 ng/ml (N=103) compared to all OvaRex^{™} patients (portrayed in Table II as a HAMA cutoff of 0 ng/ml; N=125). When HAMA responders are compared to HAMA non-responders, the increase in median time is more dramatic (see Figure 1). As Figure 1 shows, an increase in median time to relapse was observed for responders at the HAMA level of 5,000 ng/ml (N=64) and a further increase at a HAMA cut-off of 40,000 ng/ml (N=16).

The levels of HAXA (*e.g.*, HAMA is the human is being administered a murine antibody) produced in response to xenotypic antibody administration also correlate strongly with the levels of anti-idiotypic antibody (i.e., Ab2) produced in response to xenotypic antibody administration Thus, in a second aspect, the invention further provides a method for diagnosing the efficacy of xenotypic antibody-mediated immunotherapy, the method comprising measuring the level of anti-idiotype antibody (Ab2) produced in response to xenotypic antibody administration.
Preferably, Ab2 levels are at least 50 ng/ml. All other definitions and preferred embodiments are as described for the first aspect of the invention.

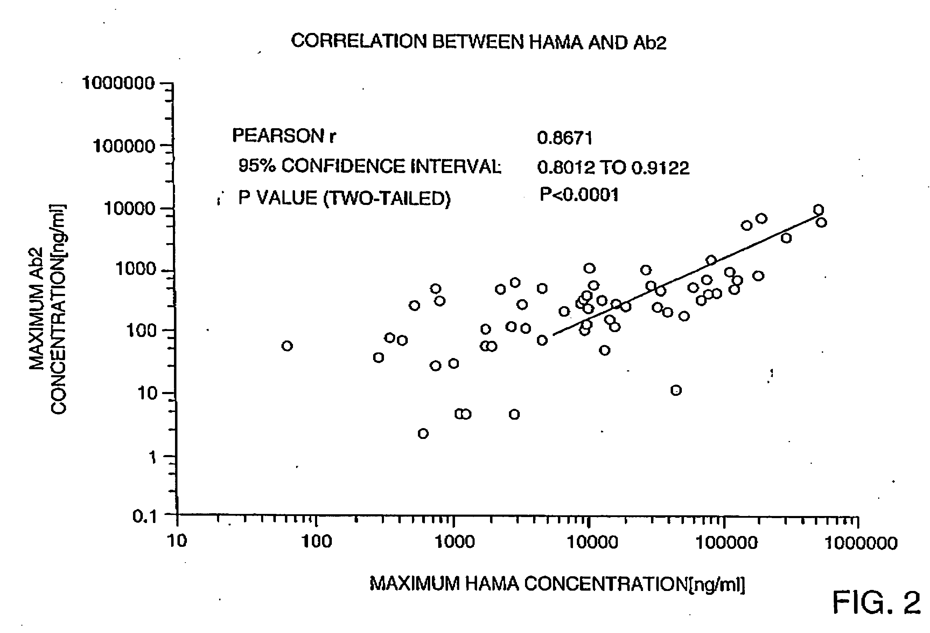

Levels of Ab2 present in patient sera were measured and compared to the levels of the HAMA response. The results are shown in Figure 2. These results demonstrate that a strong positive correlation exists between the levels of Ab2 and the level of the HAMA response.

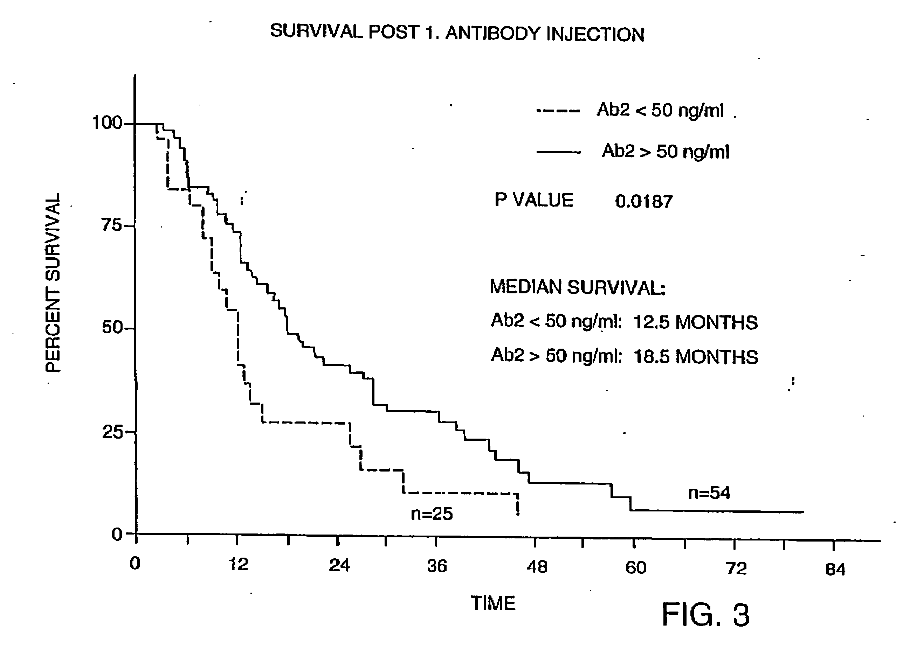

Survival time was also compared with the levels of Ab2 in these patients. The results are shown in Figure 3. The 54 patients having post-treatment Ab2 levels above 50 ng/ml had a mean survival time of 18.5 months. The 25 patients having post-treatment Ab2 levels under 50 ng/ml had a mean survival time of 125 months. These results demonstrate that post-treatment Ab2 levels are predictive of survival time. Moreover, these results indicate that if a patient produces increased levels of anti-idiotype antibody following administration of a murine monoclonal antibody, the patient has an increased survival time.

The levels of HAXA produced in response to xenotypic antibody administration also correlate strongly with the levels of antibody to the target antigen of the xenotypic antibody produced in response to xenotypic antibody administration. Thus, in a third aspect, the invention further provides a method for diagnosing the efficacy of xenotypic antibody-mediated immunotherapy, the method comprising measuring the level of antibody to the target antigen of the xenotypic antibody produced in response to xenotypic antibody administration "The target antigen of the xenotypic antibody" means an antigen in the body with which the xenotypic antibody forms an antibody-antigen binding pair. All other definitions and preferred embodiments are as described for the first aspect of the invention. Preferably the level of antibody to the target antigen of the xenotypic antibody is at least 3-fold higher than before the administration of the xenotypic antibody.

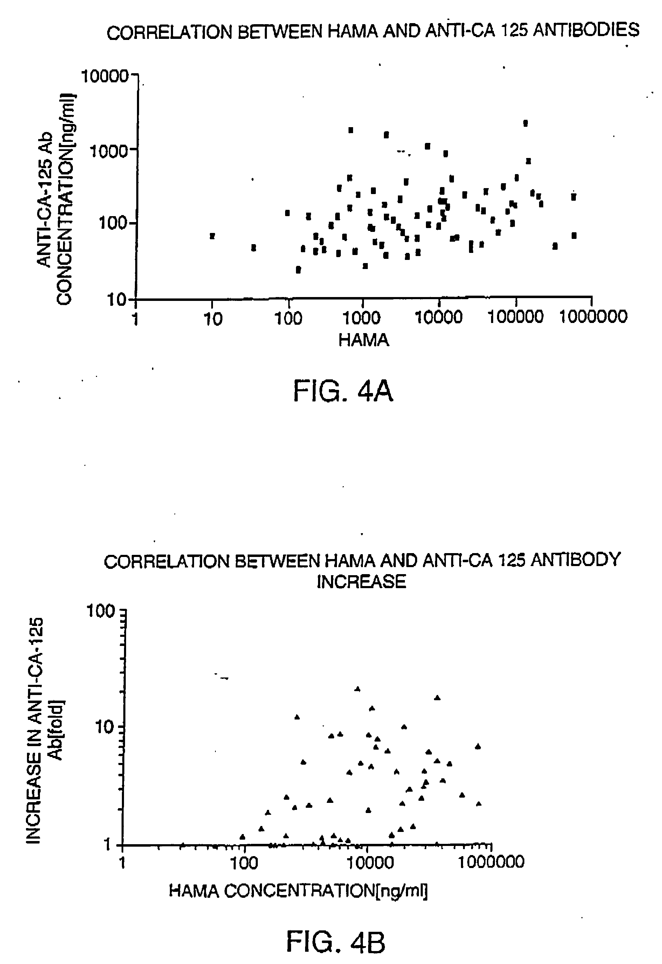

The level of antibody to the target antigen of the xenotypic antibody was compared with the level of the HAMA response. Thus, the levels of anti-CA125 antibodies produced by the patients in the above-described study were measured and compared to the level of the HAMA response of these patients. As shown in Figures 4A and 4B, as the concentration of HAMA increased, so did the patients' anti-CA125 response. These results demonstrate a strong positive correlation exists between the level of the HAMA response and the ability of the patient to generate his/her own anti-CA125 antibody.

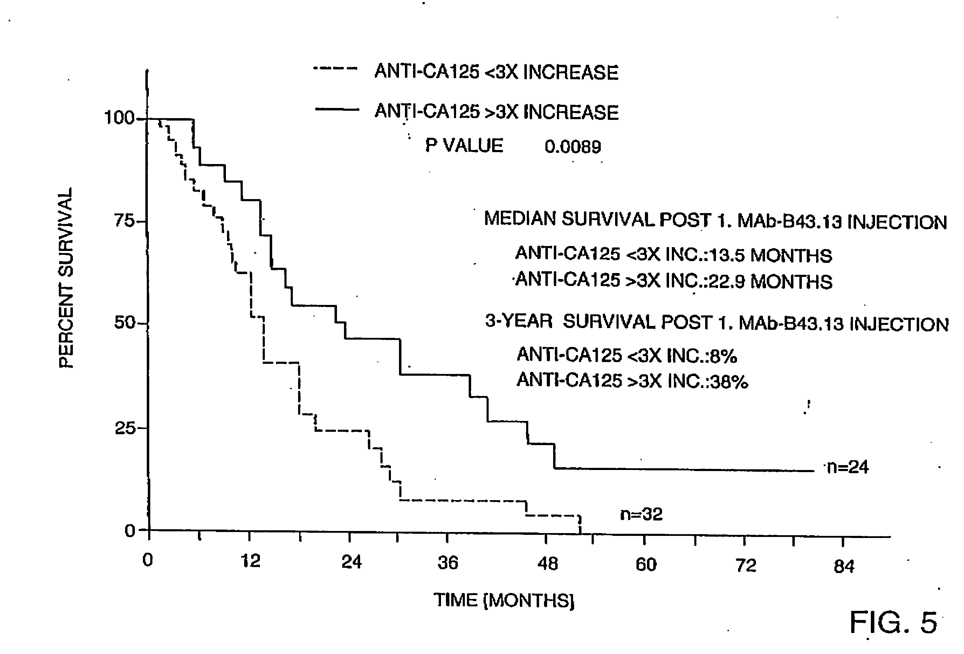

Survival time of patients was also compared with the level of antibody to the target antigen of the xenotypic antibody. The results are shown in Figure 5. The three year post-treatment survival for patients having at least a 3-fold increase in the level of antibody to the target antigen of the xenotypic antibody (as compared to the level prior to administration of the xenotypic antibody) was 38%. The three year post-treatment survival for patients having less than a 3-fold increase in the level of antibody to the target antigen of the xenotypic antibody (as compared to the level prior to administration of the xenotypic antibody) was 8%. These results demonstrate that the level of antibody to the target antigen of the xenotypic antibody is predictive of survival.

In another aspect, the invention provides a method for diagnosing the efficacy of xenotypic antibody-mediated immunotherapy, the method comprising measuring the level of T cell stimulation to the target antigen of the xenotypic antibody produced in response to xenotypic antibody administration. The term "level of T cell stimulation to the target antigen of the xenotypic antibody" means the stimulation index of T cells specific for the target antigen of the xenotypic antibody. T cell stimulation can be determined, for example, by incubating patient cells in vitro with the target antigen (*e.g.*, CA125 antigen) or tissue culture media only *(i.e.,* no antigen), pulsing the cells with ³H-thymidine, and counting the amount of ³H uptake by the cells. In this assay, the stimulation index is a comparison of the amount of ³H taken up by cells in the presence of target antigen versus the amount of ³H taken up by cells in the absence of antigen. Another method for measuring T cell stimulation (*e.g.*, for cytotoxic T cell stimulation) is a ⁵¹Cr release assay, where target cells (i.e., MHC matched) are incubated with the target antigen or tissue culture media only (*i.e.,* no antigen), and then pulsed with ⁵¹Cr. Next, patients' cells are added and the mixture of cells incubated for an amount of time, and then the amount of ⁵¹Cr released by lysed cells is measured. In this assay, the stimulation index is a comparison of the ⁵¹Cr released by cells in the presence of target antigen versus the amount of ⁵¹Cr released by the cells in the absence of antigen.

All other definitions and preferred embodiments are as described for the first aspect of the invention. Preferably, the level of T cell stimulation to the target antigen of the xenotypic antibody is at least 1.5-fold higher than before administration of the xenotypic antibody.

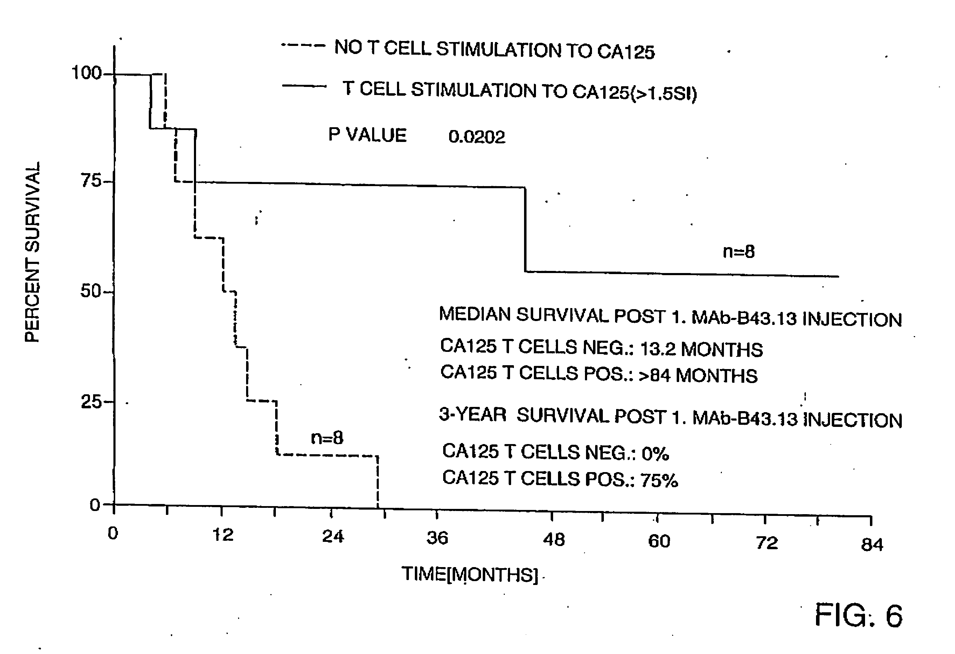

Survival time was compared with the level of T cell stimulation to the target antigen of the xenotypic antibody. The results are shown in Figure 6. Median survival time for patients having at least a 1.5-fold increase in T cell stimulation index was 84 months. Three year survival in these patients was 75%. Median survival time for patients having less than a 1.5-fold increase in T cell stimulation index was 13.2 months. Three year survival in these patients was 0%.

The following example is provided to further illustrate certain preferred embodiments of the invention and is not to be construed as narrowing the scope of the invention.

### Example 1

### Determination of HAMA response

A determination of the HAMA response of a patient may be made by using any of the numerous methods for determining an anti-murine responsive antibody concentration known to those of skill in the art of the invention. For example, any standard immunological assay, including, without limitation, ELISA or RIA, may be used to determine the HAMA response of a patient receiving treatment with the murine antibody of the invention. Such standard immunological assays are described, for example, in Ausubel et al. (1999) Current Protocols in Molecular Biology, John Wiley & Sons, New York, NY; and Coligan et al. (1999) Current Protocols in Immunology, John Wiley & Sons, New York, NY.

In one non-limiting example, a group of human patients is administered a murine antibody according to the methods of the invention. At various time points following administration of the murine antibody according to the invention, a blood sample from each patient is collected and measured for the amount of antibody present in the sample that is responsive to a murine antibody, such as the murine antibody that is used for administration. The amount of human antibody reactive to the murine antibody (i.e., the amount of the HAMA response) of each patient may be easily measured.

For example, using an EILISA-based assay to titer the HAMA response, an amount of murine antibody is used to coat the bottom of the wells in a 96 well plate. Limiting dilutions of each patient's blood sample are added to the wells of the plate, and under conditions such that the antibody in the patients' blood can specifically bind to the murine antibody.

Following antibody-specific binding, the plate is rinsed, such that the human antibody that did not specifically bind to the murine antibody coated onto the 96 well plate is removed. Next, a secondary anti-human antibody is added to each plate, and under conditions such that antibody-specific binding may occur. Preferably, the anti human antibody is labeled with a fluorophore, such that bound secondary antibody can be detected using a 96 well plate reader. The amount of HAMA activity in the patient's blood can be readily determined by determining the binding of of secondary antibody to the 96 well plate.

Whether or not the patients' blood includes Ab3 antibody (i.e., antibody produced by the patient that specifically binds to the target antigen) can be similarly determined by ELISA by determining whether the antibody in the patients' sera binds to a 96 well coated with the target antigen. Secondary anti-human antibody binding to the plate indicates the patients' are able to generate an Ab3 response following administration of a xenotypic antibody that specifically binds to the target antigen.

Whether or not the patients' blood includes T cells (helper and/or cytotoxic) that specifically bind to the target antigen in context of matched MHC can be readily determined by a helper T cell assay (*e.g.,* ³H thymidine uptake assay) or a cytotoxic T cell assay *(e.g.,* a ⁵¹Cr release assay) using MHC matched target cells *(e.g.,* from the patient him/herself) incubated with the target antigen or no antigen (negative control). Any increased proliferation by helper T cells or increased lysis by cytotoxic T cells in the presence of target antigen as compared to no antigen is indicative that the patient has a helper and/or cytotoxic T cell response.

### EQUIVALENTS

As will be apparent to those skilled in the art to which the invention pertains, the present invention may be embodied in forms other than those specifically disclosed above without departing from the spirit or essential characteristics of the invention. The particular embodiments of the invention described above, are, therefore, to be considered as illustrative and not restrictive. The patent and scientific literature referred to herein establishes knowledge that is available to those with skill in the art, and. The issued U.S. patents, allowed applications, published foreign applications, and references, including GenBank database sequences, that are cited herein are hereby incorporated by reference to the same extent as if each was specifically and individually indicated to be incorporated by reference. Any conflict between the literature cited herein and the present specification shall be resolved in favor of the latter. The scope of the invention is as set forth in the appended claims rather than being limited to the examples contained in the foregoing description.

### Clauses

1. A method for diagnosing the efficacy of xenotypic antibody-mediated immunotherapy comprising measuring the level of an antibody produced by a patient that specifically binds to a a xenotypic antibody after administration of the xenotypic antibody to the patient, wherein an increase in the level of the antibody produced by the patient after the administration of the xenotypic antibody relative to the level of antibody produced by the patient prior to the administration of the xenotypic antibody is indicative of a favorable diagnosis of efficacy.
2. The method of clause 1, wherein the level of human anti-xenotypic antibody is increased by more than two-fold relative to the level present in the patient prior to the administration of the xenotypic antibody.
3. The method of clause 1, wherein the xenotypic antibody is a murine monoclonal antibody.
4. The method of clause 1, wherein the xenotypic antibody is selected from the an antibody that specifically binds to an antigen, wherein the antigen is selected from the group consisting of CA125, MUC-1, and prostate specific antigen.
5. The method of clause 1, wherein the level of human anti-xenotypic antibody produced by a patient after administration of the xenotypic antibody to the patient is greater than or equal to 5,000 ng antibody/ml blood.
6. The method of clause 1, wherein the level of human anti-xenotypic antibody produced by a patient after administration of the xenotypic antibody to the patient is sufficient for the patient to produce an antibody that can compete with the xenotypic antibody for binding to its target antigen.
7. The method of clause 1, wherein the favorable diagnosis of efficacy increases the time to disease progression.
8. The method of clause 1, wherein the favorable diagnosis of efficacy increases the likelihood of survival of the patient.
9. The method of clause 1, wherein the patient is suffering from a disease selected from the group consisting of cancer, inflammatory disease, bacterial infection, parasitic infection, and viral infection.
10. The method of clause 1, wherein the patient is suffering from cancer.
11. The method of clause 1, wherein the patient is human.
12. A method for diagnosing the efficacy of xenotypic antibody-mediated immunotherapy comprising measuring the level of an anti-idiotype antibody produced by a patient that specifically binds to a a xenotypic antibody after administration of the xenotypic antibody to the patient, wherein an increase in the level of the anti-idiotype antibody produced by the patient after the administration of the xenotypic antibody relative to the level of anti-idiotype antibody produced by the patient prior to the administration of the xenotypic antibody is indicative of a favorable diagnosis of efficacy.
13. The method of clause 12, wherein the patient is human.
14. The method of clause 12, wherein the patient is suffering from a disease selected from the group consisting of cancer, inflammatory disease, bacterial infection, parasitic infection, and viral infection.
15. The method of clause 12, wherein the xenotypic antibody is selected from the an antibody that specifically binds to an antigen, wherein the antigen is selected from the group consisting of CA125, MUC-1, and prostate specific antigen.
16. The method of clause 12, wherein the level of antibody produced by the patient is at least 50 ng/mL blood.
17. A method for diagnosing the efficacy of xenotypic antibody-mediated immunotherapy comprising measuring the level of an antibody produced by a patient that specifically binds to a target antigen of a xenotypic antibody after administration of a xenotypic antibody to the patient, wherein an increase in the level of the antibody produced by the patient after the administration of the xenotypic antibody relative to the level of antibody produced by the patient prior to the administration of the xenotypic antibody is indicative of a favorable diagnosis of efficacy.
18. The method of clause 17, wherein the antibody produced by the patient competes with the xenotypic antibody for its binding site on the target antigen.
19. The method of clause 17, wherein the level of antibody produced by the patient after administration of the xenotypic antibody is increased by more than three-fold relative to the level present in the patient prior to the administration of the xenotypic antibody.
20. The method of clause 17, wherein the patient is human.
21. The method of clause 17, wherein the xenotypic antibody is selected from the an antibody that specifically binds to an antigen, wherein the antigen is selected from the group consisting of CA125, MUC-1, and prostate specific antigen.
22. A method for diagnosing the efficacy of xenotypic antibody-mediated immunotherapy comprising measuring the level of a T cell response produced by a patient to a target antigen of the xenotypic antibody after administration of a xenotypic antibody to the patient, wherein an increase in the level of the T cell response produced by the patient after the administration of the xenotypic antibody relative to the level of the T cell response produced by the patient prior to the administration of the xenotypic antibody is indicative of a favorable diagnosis of efficacy.
23. The method of clause 22, wherein the T cell response is a T helper cell response.
24. The method of clause 22, wherein the T helper cell response is a cytotoxic T cell response.
25. The method of clause 22, wherein the patient is human.

## Claims

1. A method of determining whether to continue to provide xenotypic antibody-mediated immunotherapy to a patient who has a disease associated with a target antigen to which the xenotypic antibody binds comprising measuring *in vitro* the level of an antibody produced by a patient that specifically binds to said xenotypic antibody before and after administration of the xenotypic antibody to the patient, wherein an increase of more than two-fold in the level of the antibody produced by the patient after the administration of the xenotypic antibody relative to the level of antibody produced by the patient prior to the administration of the xenotypic antibody identifies such patient as a target for continuing said xenotypic antibody-mediated immunotherapy.

2. The method of claim 1, wherein the xenotypic antibody is a murine monoclonal antibody.

3. The method of claim 1, wherein the xenotypic antibody binds to an antigen selected from CA125, MUC-1, and prostate specific antigen.

4. The method of claim 1, wherein the level of anti-xenotypic antibody produced by the patient after administration of the xenotypic antibody to the patient is greater than or equal to 5,000 ng antibody/ml blood.

5. The method of claim 1, wherein the level of anti-xenotypic antibody produced by a patient after administration of the xenotypic antibody to the patient is sufficient to compete with the xenotypic antibody for binding to its target antigen.

6. The method of claim 1, wherein the patient is suffering from a disease selected from cancer, an inflammatory disease, a bacterial infection, a parasitic infection, and a viral infection.

7. The method of claim 1, wherein the patient is suffering from cancer.

8. The method of claim 1, wherein the patient is human.

9. The method according to claim 1 wherein the increase is:
1) more than 3-fold the level that was present before the administration of xenotypic antibody within two weeks of administration of said xenotypic antibody; or
2) more than 3-fold higher than before administration of the xenotypic antibody.
